# EUROPEAN PATENT SPECIFICATION

(11) **EP 2 139 391 B1**
(45) Date of publication and mention of the grant of the patent: **18.05.2011**
(21) Application number: 08730689.0
(22) Date of filing: 26.02.2008
(51) Int. Cl.: A61B 5/05, A61B 19/00

(54) **RECOGNIZING A REAL WORLD FIDUCIAL IN PATIENT IMAGE DATA**
ERKENNUNG EINER REALEN STRICHMARKE IN PATIENTENBILDDATEN
RECONNAISSANCE D'UN REPÈRE RÉEL DANS DES DONNÉES IMAGE DE PATIENT

(30) Priority: 07.03.2007 US 893454 P; 21.03.2007 US 726257; 19.06.2007 US 820354; 22.08.2007 US 894841; 10.09.2007 US 852757; 10.09.2007 US 852750; 10.09.2007 US 852742; 10.09.2007 US 852728
(43) Date of publication of application: 06.01.2010
(73) Proprietor: Ethicon Endo-Surgery, Inc., Cincinnati, OH 45242 (US)
(72) Inventor: RITCHIE, Paul G., Loveland, Ohio 45140 (US); SPEEG, Trevor W.v., Williamsburg, Ohio 451 (US); DIETZ, Timothy G., Terrace Part, Ohio 45174 (US); HIBNER, John A., Mason, Ohio 45040 (US)
(74) Representative: Tunstall, Christopher Stephen
(86) International application number: PCT/US2008/054933
(87) International publication number: WO 2008/109284

(56) References cited:
- EP-A1- 1 652 470
- EP-A2- 0 920 838
- FR-A1- 2 779 339
- US-A1- 2005 085 793
- US-A1- 2006 271 056
- US-B1- 6 381 485
- VIERGEVER M.A. ET AL.: 'Integration of functional and anatomical brain images' BIOPHY. CHEM. vol. 68, 1997, pages 207 - 219, XP008119369

## Description

**Field of the Invention**

The present invention is related generally to medical images, and more particularly to medical apparatus and to a storage medium containing a computer program all relating to recognizing a real world fiducial in image data of a patient.

**Background of the Invention**

Imagers are known for obtaining image data of a patient and for displaying images of the image data on a display monitor. Such images include, without limitation, ultrasound images, X-ray images, computerized tomography (CT) images, positive electron emission (PET) images, magnetic resonance (MRI) images, fluoroscope images, etc. Where needed, it is known to register these images with a real world object by placing a fiducial component on the skin of the patient, wherein the fiducial component has a predetermined shape, and wherein the fiducial component is recognizable as a fiducial in the image data using pattern recognition software (e.g., a conventional segmentation subroutine).

Position sensors are known which are placed on medical instruments which are inserted into a patient allowing the position of the medical instrument to be tracked inside the patient. Such position sensors are part of known position sensing systems such as an AC-based system available from Biosense-Webster or a DC-based system available from Ascension Technology Corporation.

Still, scientists and engineers continue to seek improvements in recognizing a real world fiducial in patient image data.
EP 1 652 470 discloses a probe positioning technology, and an optical bioinstrumentation comprising a region selecting unit that is used to delineate a region of interest in an anatomical image of a subject, a computing unit that determines a recommended probe position according to the region of interest, a probe position sensor that detects a current probe position, a computing unit that calculates the distance between the recommended probe position and the current probe position, and an alarm device that generates an alarm sound or the like when the distance falls within a predetermined range.
US 2006/271056 discloses an osteotome instrument for use in computer assisted surgery. The instrument includes a shaft, a connector, a handle, and a cutter component. The handle has a proximal end portion and a distal end portion. The cutter component is connected to the handle at the distal end portion. The connector is releasably connected to the handle at the proximal end portion, and the connector is adapted to rotate about the shaft relative to the handle. A fiducial for tracking is connected to the connector.
US 2005/085793 discloses an apparatus having an insertable portion for holding a position sensor. The position sensor can transmit a signal indicative of its position with respect to a field generator. The insertable portion of the catheter has fiducial markings that are detectable by an imaging modality when the insertable portion is inserted into the anatomical body. The fiducial markings are asymmetrical about at least a first axis of the insertable portion. After the insertable portion has been inserted into the anatomical body, the fiducial markings can be detected to facilitate registration of the position sensor held in the insertable portion to the anatomical body. The apparatus also has a fixing mechanism for releasably fixing the insertable portion to the anatomical body. The non-symmetrical fiducial markings facilitate unambiguous registration of the insertable portion to the anatomical body. When the insertable portion is inserted into the anatomical body to a location of interest, the fixing mechanism substantially rigidly fixes the insertable portion of the catheter to a part of the anatomical body near the location of interest.

### Summary

The invention is defined by the independent claims. Optional features are included in the dependent claims.

Several benefits and advantages are obtained from one or more of the embodiments of the invention. In one example, image data is related to the fiducial component, the fiducial component is related to the position of the position sensor, and the position of the position sensor is related to a reference coordinate system allowing the creation of an image representation of the image data registered to the reference coordinate system and a display of an image of the image representation. In one variation, the fiducial component is attachable to the skin surface of the patient, the image data is obtained, and the fiducial component is removed and reattached to the same skin surface days later after which the image representation is created when the position of the attached/disposed sensor is indexed to the reference coordinate system and an image of the image representation is displayed and used while medically treating the patient. In one medical treatment, a medical instrument has its own position sensor, and an image of at least a part of the medical instrument is created and displayed superimposed on the image of the patient. An example of the third embodiment can be similarly employed.

**Brief Description of the Figures**

Figure 1 is a schematic view of a first embodiment of the invention showing medical apparatus including a top planar view of a fiducial component and a position sensor (both shown in large scale), wherein the fiducial component is attached to a patient, and the position sensor is attached to the fiducial component;

Figure 2 is a schematic view a portion of figure 1 showing a top planar view of the location indicator on the patient, showing a top planar view of the fiducial component with its pin holes, and showing a bottom planar view of the position sensor with its pins, wherein the position sensor is not yet attached to the fiducial component and the fiducial component is not yet attached to the patient;

Figure 3 is a schematic view of a display monitor upon which a digital computer displays an image of an image representation of image data of the patient registered to a reference coordinate system, wherein the fiducial component of figure 1 is shown in the image;

Figure 4 is a schematic view of a second embodiment of the invention showing medical apparatus including a top planar view of a fiducial component and a position sensor (both shown in large scale), wherein the fiducial component is attached to a patient, and the position sensor is disposed adjacent the fiducial component without attachment thereto;

Figure 5 is a schematic view of a portion of figure 3 showing a top planar view of the location indicator on the patient, showing a top planar view of the fiducial component, and showing a top planar view of the position sensor, wherein the position sensor is not yet disposed adjacent the fiducial component and the fiducial component is not yet attached to the patient;

Figure 6 is a schematic view of a display monitor upon which a digital computer displays an image of an image representation of image data of the patient registered to a reference coordinate system, wherein the fiducial component of figure 4 is shown in the image;

Figure 7 is a schematic view of a third embodiment of the invention showing medical apparatus including a top planar view of a position sensor, wherein the position sensor is attached to a patient;

Figure 8 is a schematic view a portion of figure 7 showing a top planar view of the location indicator on the patient and showing a top planar view of the position sensor, wherein the position sensor is not yet attached to the patient; and

Figure 9 is a schematic view of a display monitor upon which a digital computer displays an image of an image representation of image data of the patient registered to a reference coordinate system, wherein the position sensor of figure 7 is shown in the image.

**Detailed Description**

Before explaining the several embodiments of the present invention in detail, it should be noted that the present invention is not limited in its application or use to the details of construction and arrangement of parts and steps illustrated in the accompanying drawings and description. The illustrative embodiments of the invention may be implemented or incorporated in other embodiments, methods, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention.

It is further understood that any one or more of the following-described implementations, examples etc. can be combined with any one or more of the other following-described implementations, examples etc.

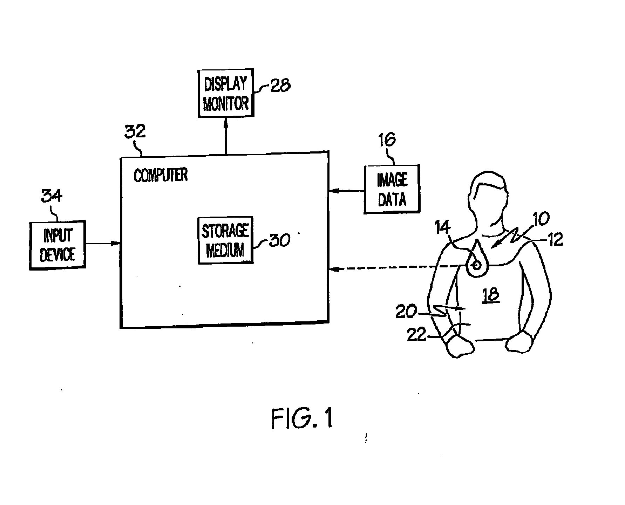

A first embodiment of the invention is shown in figures 1-3. A first expression of the embodiment of figures 1-3 is for medical apparatus 10 including a fiducial component 12 and a position sensor 14. The fiducial component 12 is recognizable as at least a part of a fiducial when appearing in image data 16 of a patient 18. The fiducial component 12 is attachable to the patient 18. The position sensor 14 is adapted to provide position data. The position sensor 14 is attachable to the fiducial component 12 at a predetermined location on the fiducial component 12 and with a predetermined orientation with respect to the fiducial component 12. It is noted that the fiducial component 12 has a predetermined shape which is recognizable as a "manufactured" shape as opposed to biological shapes occurring in image data of a patient. It is also noted that the position sensor 14 may be a wired or wireless sensor.

Examples of position sensors 14 adapted to provide position data include, without limitation, the position sensors of the AC-based position sensing system available from Biosense-Webster and the DC-based position sensing system available from Ascension Technology Corporation. It is noted that, as used in describing the embodiment of figures 1-3, the term "position" includes up to six degrees of freedom so that calculating position includes calculating a two-dimensional or three-dimensional location (translation) and two or three degrees of orientation (alignment) of the sensor 14 with respect to a reference coordinate system. A description of the operation of an embodiment of a position sensor 14 adapted to provide position data is found in US Patent Application Publication 2006/0089624.

Examples of image data 16 include, without limitation, ultrasound image data, X-ray image data, computerized tomography (CT) image data, positive electron emission (PET) image data, magnetic resonance (MRI) image data, and fluoroscope image data. An example of a computer program which creates a manipulative 3D display image from 2D CT-scans and MRI-scans is Mimics available from Materialise of Ann Arbor, Michigan.

In one enablement of the embodiment of figures 1-3, the position sensor 14 is attached to the fiducial component 12. In one variation, the attachment is a temporary attachment meaning that the position sensor 14 can be detached from the fiducial component 12 without damage to either or both of the position sensor 14 and the fiducial component 12. In another variation, the attachment is a permanent attachment meaning that the position sensor 14 cannot be detached from the fiducial component 12 without damage to either or both of the position sensor 14 and the fiducial component 12.

In one implementation of the embodiment of figures 1-3, the fiducial component 12 is recognizable as the fiducial. In one variation a portion of the overall shape of the fiducial component 12 is recognizable as the fiducial. In another variation, the overall shape of the fiducial component 12 is recognizable as the fiducial. In another implementation, it is the presence together of the fiducial component 12 (or a portion or portions thereof) and the position sensor 14 (or a portion or portions thereof) which is recognizable as the fiducial. In either or both implementations, separate portions may be recognized as separate fiducials.

In a first arrangement of the embodiment of figures 1-3, the patient 18 has skin 20 , wherein the skin 20 has a skin surface 22, wherein the fiducial component 12 is attachable to the skin surface 22 without piercing the skin surface 22, and wherein the position sensor 14 is attachable to the fiducial component 12 without piercing the skin surface 22. In one variation, the fiducial component 12 is adhesively attached to the skin surface 22. In the same or a different variation, the position sensor 14 has pins 15, the fiducial component 12 has pin holes 13, and the pins 15 are attachingly engagable with the pin holes 13. In one modification, not shown, the position sensor has a sensor body and a sensor, wherein the sensor body has a sensor location and rotational orientation feature such that the sensor is attachable to the sensor body only at the sensor location and with the rotational orientation. Other attachment variations and other modifications are left to the artisan.

A first method of the invention is for using the first arrangement of the medical apparatus 10 and includes steps a) through g). Step a) includes placing a location indicator 24 on the skin surface 22 where the fiducial component 12 is to be attached to the skin surface 22, wherein the location indicator 24 also indicates a desired orientation of the fiducial component 12 on the skin surface 22. Step b) includes, after step a), attaching the fiducial component 12 to the skin surface 22 at the location indicator 24 and with the desired orientation. Step c) includes, after step b), obtaining image data 16 of the patient 18, wherein the fiducial component 12 appears in the image data 16. Step d) includes, after step c), removing the fiducial component 12 from the skin surface 22 while leaving the location indicator 24 on the skin surface 22. Step e) includes, at a later time after steps a) through d), re-attaching the fiducial component 12 to the skin surface 22 at the location indicator 24 and with the desired orientation. Step f) includes, after step e), creating an image representation of the image data 16 indexed to a reference coordinate system using at least the recognized predetermined shape and a position of the position sensor 14 indexed to the reference coordinate system when the position sensor 14 is attached to the re-attached fiducial component 12. Step g) includes, after step f), displaying an image 26 of the image representation. It is noted that step c) may be performed with or without the position sensor 14 attached to the fiducial component 12.

In one realization of the first method, the position data and/or the image data 16 are already indexed and step e) does not perform such indexing. In a different realization of the first method, the position data and/or the image data 16 are not yet indexed, and step e) performs such indexing.

In one illustration of the first method, the position sensor 14 is considered to be a position sensor of a Biosense Webster positioning sensing system and a transmitter, not shown, of such system is used by a digital computer for a reference coordinate system for position data from the position sensor 14. Thus, the position of the position sensor 14 can be indexed to the reference coordinate system. Since the image data 16 is related to the fiducial component 12 which has a predetermined position with respect to the attached position sensor 14, an image representation of the image 26 can be created which is registered to the reference coordinate system.

In one employment of the first method, step g) displays the image 26 on a display monitor 28. Examples of a display monitor 42 include, without limitation, a computer monitor, a goggle display screen, and a room wall upon which projected images are displayed. In one variation, a storage medium 30 contains a program readable by a digital computer 32 which instructs the digital computer 32 to perform steps f) and g) of the first method.

In one example, the image 26 is a three-dimensional manipulative image, and there is also included a computer input device 34 operatively connected to the digital computer 32 to allow a user to manipulate the three-dimensional-manipulative image on the display monitor 28. Examples of input devices 34 include, without limitation, a keyboard and a mouse. In a different example, the image is a two-dimensional non-manipulative image.

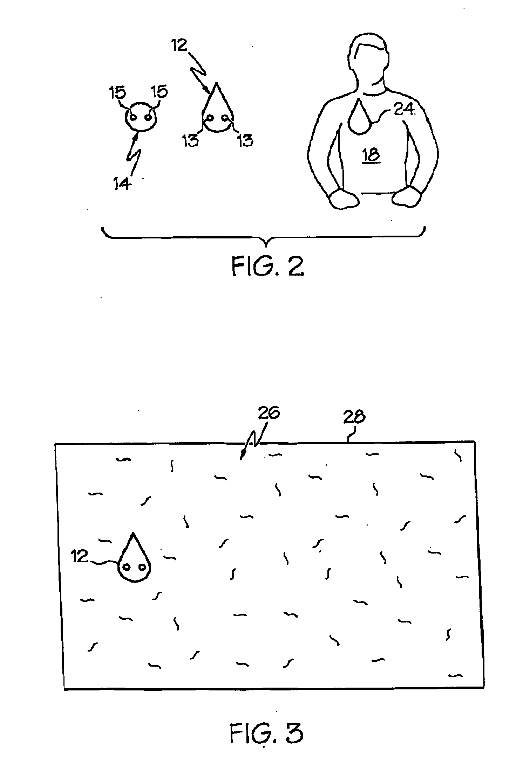

In one variation of the first method, step e) is performed at least 24 hours after performing steps a) through d). In the same or a different variation, the location indicator 24 is an invisible ink outline of the fiducial component 12 on the skin surface 22 (such as the ultraviolet-ink outline made visible under ultraviolet light as seen in figure 2) or is a clear adhesive decal outline of the fiducial component on the skin surface. In one modification, the outline is asymmetric and matches the asymmetric shape of the fiducial component 12 for proper location and alignment of the fiducial component 12 on the skin surface 22. A medical treatment (such as a surgical treatment) of the patient 18 could be performed while viewing the displayed image 26. In one medical treatment, not shown, a medical instrument has its own position sensor, and an image of at least a part of the medical instrument is created and displayed superimposed on the image 26 of the patient 18. Other variations and modifications are left to the artisan including performing step e) at least 15 minutes after performing steps a) through d) or performing step e) at a shorter or longer time interval after performing steps a) through d). In one example, without limitation, a person who has had MRI or CT images taken in an imaging area of a medical facility where steps a) through d) were performed is then quickly moved to a surgical area of the medical facility where steps e) through g) are performed.

In a second arrangement of the embodiment of figures 1-3, not shown, the fiducial component is attachable to an internal skeletal feature of the patient. In a third arrangement, not shown, the fiducial component is attachable to the patient below the skin of the patient, and the position sensor has at least one portion adapted for piercing the skin for attaching to the fiducial component. In a fourth arrangement, the fiducial component is attachable to the patient within the skin of the patient, and the position sensor has at least one portion adapted for piercing the skin surface for attaching to the fiducial component. In a fifth arrangement, the fiducial component is attachable to orthopaedic hardware of the patient.

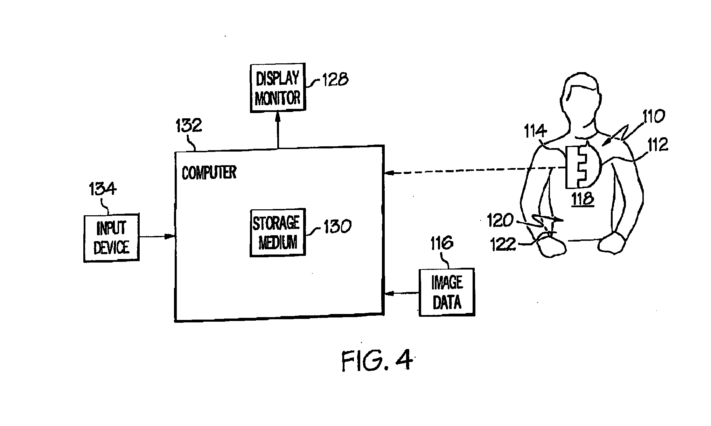

A second embodiment of the invention is shown in figures 4-6. A first expression of the embodiment of figures 4-6 is for a medical apparatus 110 including a fiducial component 112 and a position sensor 114. The fiducial component 112 is recognizable as at least a part of a fiducial when appearing in image data 116 of a patient 118. The fiducial component 112 is attachable to the patient 118. The position sensor 14 is adapted to provide position data. The position sensor 114 is disposable adjacent the fiducial component 112 without attachment thereto at a predetermined location on the fiducial component 112 and with a predetermined orientation with respect to the fiducial component 112. It is noted that the fiducial component 112 has a predetermined shape which is recognizable as a "manufactured" shape as opposed to biological shapes occurring in image data of a patient.

In one enablement of the embodiment of figures 4-6, the position sensor 114 is disposed adjacent the fiducial component 112 without attachment thereto at the predetermined location on the fiducial component 112 and with the predetermined orientation with respect to the fiducial component 112.

In one implementation of the embodiment of figures 4-6, the fiducial component 112 is recognizable as the fiducial.

In a first arrangement of the embodiment of figures 4-6, the patient 118 has skin 120 , wherein the skin 120 has a skin surface 122, wherein the fiducial component 112 is attachable to the skin surface 122 without piercing the skin surface 122, and wherein the position sensor 114 is attachable to the skin surface 122 adjacent the fiducial component 112 without piercing the skin surface 122. In one variation, the fiducial component 112 and the position sensor 114 each are adhesively attached to the skin surface 122. The position sensor 114 and the fiducial component 112 each have a complementary-shaped portion which allows the position sensor 114 to be disposed adjacent the fiducial component 112 at a desired location on the fiducial component 112 and with a desired alignment with respect to the fiducial component 112.

A second method of the invention is for using the first arrangement of the medical apparatus 110 and includes steps a) through g). Step a) includes placing a location indicator 124 on the skin surface 122 where the fiducial component 112 is to be attached to the skin surface 122, wherein the location indicator 124 also indicates a desired orientation of the fiducial component 112 on the skin surface 122. Step b) includes, after step a), attaching the fiducial component 112 to the skin surface 122 at the location indicator 124 and with the desired orientation. Step c) includes, after step b), obtaining image data 116 of the patient 118, wherein the fiducial component 112 appears in the image data 116. Step d) includes, after step c), removing the fiducial component 112 from the skin surface 122 while leaving the location indicator 124 on the skin surface 122. Step e) includes, at a later time after steps a) through d), re-attaching the fiducial component 112 to the skin surface 122 at the location indicator 124 and with the desired orientation. Step f) includes, after step e), creating an image representation of the image data 116 indexed to a reference coordinate system using at least the recognized predetermined shape and a position of the position sensor 114 indexed to the reference coordinate system when the position sensor 114 is disposed adjacent the re-attached fiducial component 112. Step g) includes, after step f), displaying an image 126 of the image representation. It is noted that step c) may be performed with or without the position sensor 114 disposed adjacent (e.g., on the side or top surface of) the re-attached fiducial component 112 at the desired location on the fiducial component 112 and with the desired alignment with respect to the fiducial component 112.

In one illustration of the second method, the position sensor 114 is considered to be a position sensor of a Biosense Webster positioning sensing system and a transmitter, not shown, of such system is used by a digital computer for a reference coordinate system for position data from the position sensor 114. Thus, the position of the position sensor 114 can be indexed to the reference coordinate system. Since the image data 116 is related to the fiducial component 112 which has a predetermined position with respect to the properly adjacently disposed position sensor 114, an image representation of the image 126, can be created which is registered to the reference coordinate system.

In one employment of the second method, step g) displays the image 126 on a display monitor 128. In one variation, a storage medium 130 contains a program readable by a digital computer 132 which instructs the digital computer 132 to perform steps f) and g) of the second method.

In one example, the image 126 is a three-dimensional manipulative image, and there is also included a computer input device 134 operatively connected to the digital computer 132 to allow a user to manipulate the three-dimensional-manipulative image on the display monitor 128. In a different example, the image is a two-dimensional non-manipulative image.

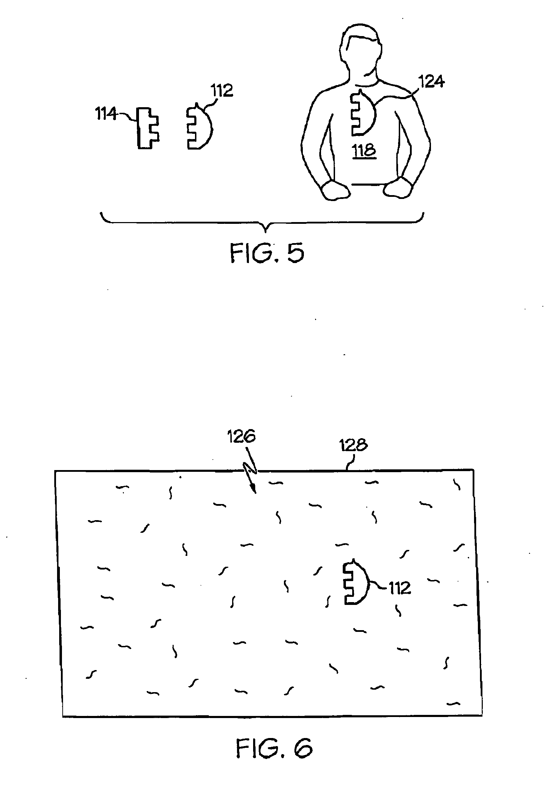

In one variation of the second method, step e) is performed at least 24 hours after performing steps a) through d). In the same or a different variation, the location indicator 124 is an invisible ink outline of the fiducial component 112 on the skin surface 122 (such as the ultraviolet-ink outline made visible under ultraviolet light as seen in figure 5) or is a clear adhesive decal outline of the fiducial component on the skin surface. The outline is asymmetric and matches the asymmetric shape of the fiducial component 112 for proper location and alignment of the fiducial component 112 on the skin surface 122. In the same or a different variation, the second method also includes the step of performing a medical treatment (such as a surgical treatment) of the patient 118 while viewing the displayed image 126. In one medical treatment, not shown, a medical instrument has its own position sensor, and an image of at least a part of the medical instrument is created and displayed superimposed on the image 126 of the patient 118.

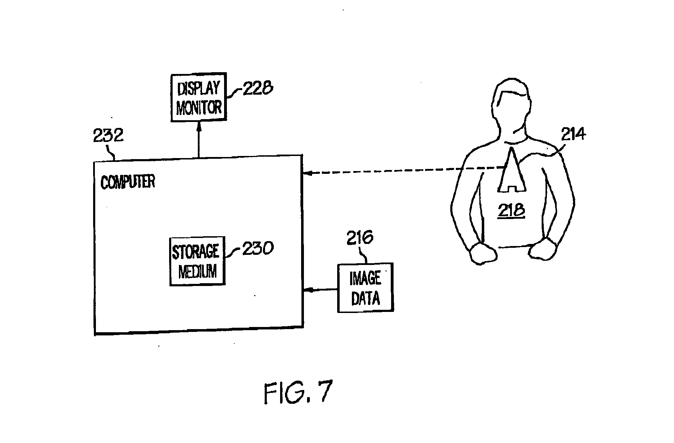

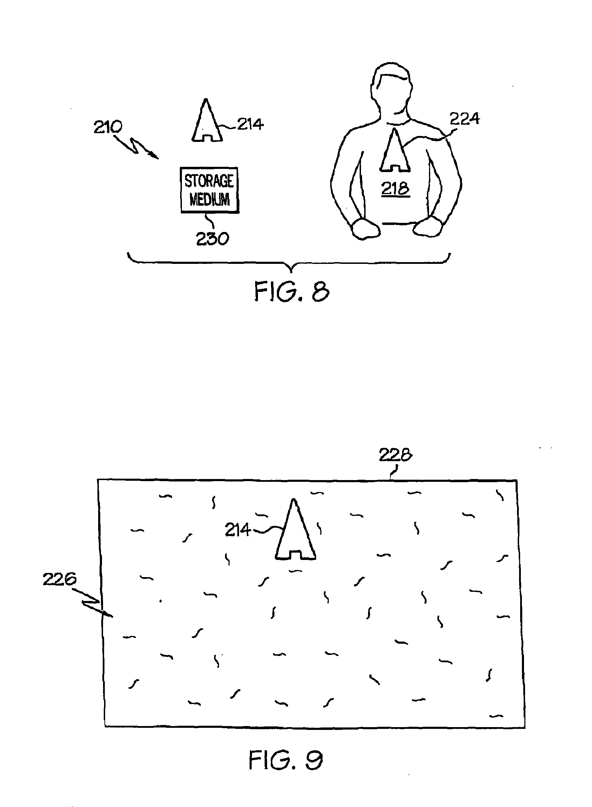

A third embodiment of the invention is shown in figures 7-9. A first expression of the embodiment of figures 7-8 is for a storage medium 230 containing a program readable by a digital computer 232 which instructs the digital computer 232 to recognize a predetermined shape of each of at least one portion of a position sensor 214 as at least a part of a real-world fiducial in image data 216 of a patient 218 when the image data 216 includes the predetermined shape and is received as an input by the digital computer 232. The position sensor 214 is adapted to provide position data. It is noted that the words "at least one portion" includes "the entirety". In one example, the program includes a conventional segmentation subroutine to identify the predetermined shape.

Examples of storage media include, without limitation, temporary computer memory and permanent computer memory such as RAM, hard drives, CD's, etc.

In one enablement of the first expression of the embodiment of figures 7-9, the at-least-one portion is adapted to have a fixed position relative to the patient 218 during a medical treatment of the patient 218. In the same or a different enablement, the program instructs the digital computer 232 to create an image representation of the image data 216 indexed to a reference coordinate system using at least the recognized predetermined shape and a position of the position sensor indexed to the reference coordinate system, and the program instructs the digital computer 232 to display an image 226 (such as on a display monitor 228) of the image representation. It is noted that code can be written by those of ordinary skill in the art, without undue experimentation, which instructs the digital computer 132 to create the image representation of the image data 116 indexed to the reference coordinate system.

In one extension of the first expression of the embodiment of figures 7-9, a location indicator 224 (similar to the previously described location indicators 24 and 124) is disposed on the patient 218, and the position sensor 214 is disposable on the patient 218 on, and alignable with, the location indicator 224.

A second expression of the embodiment of figures 7-9 is for medical apparatus 210 including a position sensor 214 and a storage medium 230. The position sensor 214 has at least one portion each with a predetermined shape. The storage medium 230 contains a program readable by a digital computer 232 which instructs the digital computer 232 to recognize the predetermined shape of each of the at-least-one portion as at least a part of a real-world fiducial in image data 216 of a patient 218 when the image data 216 includes the predetermined shape and is received as an input by the digital computer 232. The position sensor 214 is adapted to provide position data.

It is noted that the enablements, etc. of the first expression of the embodiment of figures 7-9 are equally applicable to the second expression of the embodiment of figures 7-9.

Several benefits and advantages are obtained from one or more of the embodiments of the invention. In one example of the first and/or second embodiment, image data is related to the fiducial component, the fiducial component is related to the position of the position sensor, and the position of the position sensor is related to a reference coordinate system allowing the creation of an image representation of the image data registered to the reference coordinate system and a display of an image of the image representation. In one variation, the fiducial component is attachable to the skin surface of the patient, the image data is obtained, and the fiducial component is removed and reattached to the same skin surface days later after which the image representation is created when the position of the attached/disposed sensor is indexed to the reference coordinate system and an image of the image representation is displayed and used while medically treating the patient. In one medical treatment, a medical instrument has its own position sensor, and an image of at least a part of the medical instrument is created and displayed superimposed on the image of the patient. An example of the third embodiment can be similarly employed.

While the present invention has been illustrated by several embodiments and methods, and enablements, applications, etc. thereof, it is not the intention of the applicants to restrict or limit the spirit and scope of the appended claims to such detail. Numerous other variations, changes, and substitutions will occur to those skilled in the art without departing from the scope of the invention. It will be understood that the foregoing description is provided by way of example, and that other modifications may occur to those skilled in the art without departing from the scope and spirit of the appended Claims.

## Claims

1. Medical apparatus (110) comprising a fiducial component (112) and a position sensor (114), wherein the fiducial component is recognizable as at least a part of a fiducial when appearing in image data (126) of the patient (118), wherein the fiducial component is attachable to the patient, wherein the position sensor is adapted to provide position data, and wherein the position sensor is adjoinable to the fiducial component without attachment thereto at a predetermined location on the fiducial component and with a predetermined orientation with respect to the fiducial component, wherein the position sensor and the fiducial component each have a complementary-shaped portion configured to adjoin the position sensor to the fiducial component at the predetermined location on the fiducial component and with the predetermined orientation with respect to the fiducial component, and wherein the fiducial component has an asymmetric shape, formed at least in part by its complementary-shaped portion.

2. The medical apparatus (110) of claim 1, wherein the position sensor (114) is adjoined to the fiducial component (112) without attachment thereto at the predetermined location on the fiducial component and with the predetermined orientation with respect to the fiducial component.

3. The medical apparatus (110) of claim 1, wherein the fiducial component (112) is attachable to the patient's skin surface (122) without piercing the skin surface, and wherein the position sensor (114) is attachable to the skin surface adjacent the fiducial component without piercing the skin surface.

4. A method for using the medical apparatus (110) of claim 3 comprising:
a) placing a location indicator (124) on the skin surface (122) where the fiducial component (112) is to be attached to the skin surface, wherein the location indicator also indicates a desired orientation of the fiducial component on the skin surface;
b) after step a), attaching the fiducial component to the skin surface at the location indicator and with the desired orientation;
c) after step b), obtaining image data (126) of the patient (118), wherein the fiducial component appears in the image data;
d) after step c), removing the fiducial component from the skin surface while leaving the location indicator on the skin surface; and
e) at a later time after steps a) through d), re-attaching the fiducial component to the skin surface at the location indicator and with the desired orientation;
f) after step e), creating an image representation of the image data indexed to a reference coordinate system using at least the recognized predetermined shape and a position of the position sensor (114) indexed to the reference coordinate system when the position sensor is adjoined to the re-attached fiducial component; and
g) after step f), displaying an image of the image representation.

5. The method of claim 4, wherein step e) is performed at least 24 hours after performing steps a) through d).

6. The method of claim 5, wherein the location indicator (124) is an invisible ink outline of the fiducial component (112) disposed on the skin surface (122).

7. A storage medium containing a program readable by a digital computer which instructs the digital computer to perform steps f) and g) of the method of claim 4.

8. Medical apparatus comprising the medical apparatus of claim 1 and the storage medium in accordance with claim 7.

## Patentansprüche

1. Medizinisches Gerät (110) mit einer Strichmarkenkomponente (112) und einem Positionssensor (114), worin die Strichmarkenkomponente zumindest als Teil einer Strichmarke zu erkennen ist, wenn sie in Bilddaten (126) des Patienten (118) erscheint, worin die Strichmarkenkomponente am Patienten befestigt werden kann, worin der Positionssensor Positionsdaten liefern kann und worin der Positionssensor ohne Befestigung an einer vorbestimmten Stelle auf der Strichmarkenkomponente und mit einer vorbestimmten Orientierung relativ zur Strichmarkenkomponente an die Strichmarkenkomponente angrenzen kann, worin der Positionssensor und die Strichmarkenkomponente jeweils einen komplementär geformten Abschnitt aufweisen, der so konfiguriert ist, dass er den Positionssensor mit der Strichmarkenkomponente an der vorbestimmten Stelle auf der Strichmarkenkomponente und mit der vorbestimmten Orientierung relativ zur Strichmarkenkomponente verbindet und worin die Strichmarkenkomponente eine asymmetrische Gestalt aufweist, die zumindest teilweise von ihrem komplementär geformten Abschnitt geformt wird.

2. Medizinisches Gerät (110) nach Anspruch 1, worin der Positionssensor (114) ohne Befestigung an der vorbestimmten Stelle auf der Strichmarkenkomponente und mit der vorbestimmten Orientierung relativ zur Strichmarkenkomponente an die Strichmarkenkomponente (112) angrenzt.

3. Medizinisches Gerät (110) nach Anspruch 1, worin die Strichmarkenkomponente (112) auf der Hautoberfläche (122) des Patienten befestigt werden kann, ohne die Hautoberfläche zu durchstechen, und worin der Positionssensor (114) neben der Strichmarkenkomponente auf der Hautoberfläche befestigt werden kann, ohne die Haut zu durchstechen.

4. Verfahren zur Anwendung des medizinischen Geräts (110) nach Anspruch 3, das Folgendes umfasst:
a) Platzierung eines Lokalisationsindikators (124) auf der Hautoberfläche (122), an der die Strichmarkenkomponente (112) auf der Hautoberfläche befestigt werden soll, worin der Lokalisationsindikator auch eine gewünschte Orientierung der Strichmarkenkomponente auf der Hautoberfläche anzeigt;
b) nach Schritt a) Befestigung der Strichmarkenkomponente auf der Hautoberfläche am Lokalisationsindikator und mit der gewünschten Orientierung;
c) nach Schritt b) Gewinnung von Bilddaten (126) des Patienten (118), worin die Strichmarkenkomponente in den Bilddaten erscheint;
d) nach Schritt c) Entfernung der Strichmarkenkomponente von der Hautoberfläche, während der Lokalisationsindikator auf der Hautoberfläche verbleibt; und
e) zu einem späteren Zeitpunkt nach Schritt a) bis d) erneute Befestigung der Strichmarkenkomponente auf der Hautoberfläche am Lokalisationsindikator und mit der gewünschten Orientierung;
f) nach Schritt e) Erzeugung einer Bilddarstellung der Bilddaten verbunden mit einem Bezugskoordinatensystem unter Verwendung zumindest der erkannten vorbestimmten Gestalt und einer Position des Positionssensors (114) verbunden mit dem Bezugskoordinatensystem, wenn der Positionssensor an die erneut befestigte Strichmarkenkomponente angrenzt; und
g) nach Schritt f) Anzeige eines Bildes der Bilddarstellung.

5. Verfahren nach Anspruch 4, worin Schritt e) zumindest 24 Stunden nach Schritt a) bis d) durchgeführt wird.

6. Verfahren nach Anspruch 5, worin der Lokalisationsindikator (124) ein Umriss mit unsichtbarer Tinte der Strichmarkenkomponente (112) auf der Hautoberfläche (122) ist.

7. Speichermedium, das ein von einem Digitalcomputer lesbares Programm enthält, das den Digitalcomputer anweist, Schritt f) und g) des Verfahrens nach Anspruch 4 durchzuführen.

8. Medizinisches Gerät, welches das medizinische Gerät nach Anspruch 1 und das Speichermedium nach Anspruch 7 umfasst.

## Revendications

1. Appareil (110) médical comportant un élément (112) de repérage et un capteur (114) de position, dans lequel l'élément de repérage peut être reconnu au moins en tant que partie d'un repère lorsqu'il apparaît dans des données (126) image du patient (118), dans lequel l'élément de repérage peut être fixé au patient, dans lequel le capteur de position est conçu pour fournir des données de position, et dans lequel le capteur de position peut être positionné latéralement contre l'élément de repérage sans le fixer à celui-ci à un emplacement prédéterminé sur l'élément de repérage et avec une orientation prédéterminée par rapport à l'élément de repérage, dans lequel le capteur de position et l'élément de repérage ont chacun une portion de forme complémentaire conçue pour positionner le capteur de position latéralement contre l'élément de repérage à l'emplacement prédéterminé sur l'élément de repérage et avec l'orientation prédéterminée par rapport à l'élément de repérage, et dans lequel l'élément de repérage a une forme asymétrique, constituée au moins en partie par sa portion de forme complémentaire.

2. Appareil (110) médical selon la revendication 1, dans lequel le capteur (114) de position est positionné latéralement contre l'élément (112) de repérage sans le fixer à celui-ci à l'emplacement prédéterminé sur l'élément de repérage et avec l'orientation prédéterminée par rapport à l'élément de repérage.

3. Appareil (110) médical selon la revendication 1, dans lequel l'élément (112) de repérage peut être fixé à la surface (122) de la peau du patient sans percer la surface de la peau, et dans lequel le capteur (114) de position peut être fixé à la surface de la peau à côté de l'élément de repérage sans percer la surface de la peau.

4. Procédé pour utiliser l'appareil (110) médical selon la revendication 3 comportant les étapes suivantes :
a) placer un indicateur (124) d'emplacement sur la surface (122) de la peau à l'endroit où l'élément (112) de repérage doit être fixé sur la surface de la peau, dans lequel l'indicateur d'emplacement indique aussi une orientation souhaitée de l'élément de repérage sur la surface de la peau ;
b) après l'étape a), fixer l'élément de repérage sur la surface de la peau au niveau de l'indicateur d'emplacement et avec l'orientation souhaitée ;
c) après l'étape b), obtenir des données (126) image du patient (118), dans lequel l'élément de repérage apparaît dans les données image ;
d) après l'étape c), enlever l'élément de repérage de la surface de la peau tout en laissant l'indicateur d'emplacement sur la surface de la peau ; et
e) à une étape ultérieure après les étapes a) jusqu'à d), fixer à nouveau l'élément de repérage sur la surface de la peau au niveau de l'indicateur d'emplacement et avec l'orientation souhaitée ;
f) après l'étape e), créer une représentation image des données image indexée à un système de coordonnées de référence en utilisant au moins la forme prédéterminée reconnue et une position du capteur (114) de position indexée au système de coordonnées de référence lorsque le capteur de position est positionné latéralement contre l'élément de repérage qui a été à nouveau fixé ; et
g) après l'étape f), afficher une image de la représentation image.

5. Procédé selon la revendication 4, dans lequel l'étape e) est réalisée au moins 24 heures après avoir réalisé les étapes a) jusqu'à d).

6. Procédé selon la revendication 5, dans lequel l'indicateur (124) d'emplacement est un contour à l'encre invisible de l'élément (112) de repérage disposé sur la surface (122) de la peau.

7. Support de mise en mémoire contenant un programme pouvant être lu par un ordinateur numérique qui donne l'instruction à l'ordinateur numérique de réaliser les étapes f) et g) du procédé de la revendication 4.

8. Appareil médical comportant l'appareil médical de la revendication 1 et le support de mise en mémoire selon la revendication 7.
